# EUROPEAN PATENT APPLICATION

(11) **EP 4 011 351 A1**
(43) Date of publication of application: **15.06.2022**
(21) Application number: 19940586.1
(22) Date of filing: 06.08.2019
(51) Int. Cl.: A61K 8/19, A61K 8/36, A61K 8/368, A61K 8/73, A61Q 19/00

(54) **TOPICAL COSMETIC COMPOSITION, USE OF THE COSMETIC COMPOSITION AND MASK FOR FACIAL APPLICATION**

(71) Applicant: Natura Cosméticos S.A., 05106-000 São Paulo SP (BR)
(72) Inventor: MATHIAS, Michele Helena, 07790-190 Cajamar - SP (BR); PEREIRA SANTOS, Camila, 07790-190 Cajamar - SP (BR); DE MIRANDA CHAVES VASQUEZ PINTO, Luciana, 07790-190 Cajamar - SP (BR); ARANDAS MONTEIRO E SILVA, Silas, 07790-190 Cajamar - SP (BR); SAVIETTO, Joice, 07790-190 Cajamar - SP (BR); LIMA GUSMÃO, Edjane, 07790-190 Cajamar - SP (BR); GREGÓRIO PINTO, Nicole, 07790-190 Cajamar - SP (BR)
(74) Representative: Simmons & Simmons
(86) International application number: PCT/BR2019/050321
(87) International publication number: WO 2021/022347

(57) **Abstract**

The present invention refers to a topical cosmetic composition that provides immediate and prolonged reduction and control of skin oiliness, as well as pore size reduction, reduction of skin shine and visual imperfections. The composition further maintains the skin's natural and healthy microbiota. The present invention is within the field of cosmetic science, more precisely it concerns skin care preparations.

## Description

### Field of the invention

The present invention describes a topical cosmetic composition that provides immediate and prolonged reduction and control of skin oiliness, as well as pore size reduction, reduction of skin shine and visual imperfections. The present invention is within the field of cosmetic science, more precisely it concerns skin care preparations.

### Background of the invention

Skin or integument is a double layered membrane that covers the entire body surface and is adjacent to mucous membranes that cover the human body orifices. It exhibits great variation in thickness along its entire length, ranging from 1 to 4 mm according to its required biological functions. Among these functions are maintenance of the body integrity by protecting the body against injuries, absorbing and excreting liquids, regulating the body temperature, absorbing ultraviolet rays, metabolizing vitamin D, providing sensory stimuli and cosmetic functions.

The skin can be divided into two parts, the outermost layer being represented by the epidermis and the innermost layer being the dermis which rests on the subcutaneous cellular tissue, which is also part of the skin's structure.

The epidermis is formed by a keratinized stratified squamous epithelium that contains 4 layers: basal, spinous, granular and corneal (horny) layers.

The epidermis cells, in turn, can be divided into 4 groups: stem cells, proliferative cells, differentiated cells and functional cells. The epidermis also has dendritic cells (melanocytes and Langerhans cells) and neuroendocrine (Merkel) cells. Like every epithelium, the epidermis cells are renewed indefinitely thanks to a continuous mitotic activity.

Due to exposure to the environment, the skin is covered by a lipid layer from sebum and epidermal lipids. Sebum is an important skin factor, as it can be classified according to the amount of sebum secreted. In the cosmetic field, the facial skin is usually classified as dry, normal, combination or oily skin.

There is a cosmetic concern related to oily skin, that is, skin that produces a large amount of sebum. This is because such a condition modulates skin aspects such as: intense shine, sticky feeling, tendency to acne, pimples and blackheads and dilated and irregular pores.

Sebum is a lipid mixture comprising triglycerides, fatty acid esters, esterified waxes, squalene and cholesterol esters. Its main function is to protect the skin by controlling transdermal water loss, forming a waterproof barrier.

The face has an average of three to nine hundred pilosebaceous follicles per square centimeter, however, several factors are involved in the regulation of the amount of sebum deposited on the skin surface, so what happens in the sebaceous gland should not be considered as the only cause of oiliness on the skin.

In people with oily skin, the sebaceous glands are hyperactivated, which can cause excessive shine and the presence of dilated pores. A consequence of dilated pores is an even more oily skin, with a greasy and shiny appearance.

A reduction in the pore diameter leads to a reduced amount of oil released and it further provides the skin with a healthier and blemish-free appearance.

Thus, there is a technical problem in the cosmetic field related to the need to provide alternative compositions to reduce and control oiliness of the skin, especially facial skin.

Oiliness is still related to disturbances in the skin microbiota. This is because the microbiota has a variety of microorganisms that depend on the skin conditions. Where oiliness increases due to internal or external factors, the skin conditions favor bacteria that do not require oxygen to grow and use lipids instead. Such a population increases, reducing other bacterial populations that grow better in less oily environments. Thus, the inflammatory response that leads to the appearance of acne keeps the vicious circle of excessive oil and lipid-consuming bacteria, leaving the skin unbalanced.

The microbiota (set of microorganisms that inhabit an ecosystem) is found throughout the skin, being more concentrated in warmer and moist regions, such as the armpits and perineum, reaching the number of 10 bacteria per cm². Gram positive bacteria, such as *Staphylococcus*, *Corynebacterium* and *Propionobacterium* usually predominate. It interacts with other microbes, human cells and the immune system via different pathways that mediate the risk for diseases. Disturbances in this microbiota may influence the onset of diseases.

Therefore, one of the important issues in the treatment of oily skin is to maintain a balanced microbiota and not just eliminate all existing bacteria populations, as they aid to maintain the immune system by mediating the risk for diseases.

The state of the art describes a variety of cosmetic compositions. For example, WO9503779 relates to a cosmetic composition for the treatment of pimples and redness. The cosmetic composition including at least one keratolytic agent and a combination of water-soluble and water-insoluble anti-irritanting agents in a cosmetically acceptable carrier. A combination of C7-C30 p-hydroxycarboxylic acid, such as salicylic acid with C1-C25a hydroxycarboxylic acid, such as glycolic or lactic acid, is effective in the composition. The document does not disclose mixing with clays or babassu.

WO2015030702 refers to an improvement in the cosmetic application of rhamnolipids. More specifically, it refers to the purity of higher rhamnolipids, different formulations, different application rates, and more effective carriers.

WO2013149323 refers to a natural product for skin care, protection, rehabilitation and regeneration and to maintain the skin's well-being. More particularly, it concerns an excipient combined with a lipid composition.

Accordingly, it is understood that in view of the relevant state of the art, there remains the need to provide cosmetic compositions to reduce and control oiliness of the skin in an immediate and prolonged manner while reducing the shine and pore size, masking visual imperfections of the skin.

### Summary of the invention

Thus, the present invention is intended to solve the technical issues of the state of the art by providing a topical cosmetic composition that reduces and controls oiliness of the skin in an immediate and prolonged manner, as well as reduces the pore size, the skin shine, and masks optical imperfections. The composition further maintains the skin's natural and healthy microbiota.

In a first aspect the present invention relates to a topical cosmetic composition in the form of a clay face mask, wherein the composition comprises: clay, mandelic acid, salicylic acid and babassu starch.

In a second aspect, the present invention relates to the use of the composition to reduce and control skin oiliness.

In a third aspect, the present invention relates to the use of the composition to reduce the average size of skin pores.

In a forth aspect, the present invention relates to the use of the composition to reduce visual skin imperfections.

In a fourth aspect the present invention relates to the use of the composition to maintain the skin.

Also, the inventive concept in common to all aspects of the invention is that they involve the composition of the first aspect.

From among the advantages of the present invention, in a non-exhaustive manner, is that it is able to:
- the invention provides immediate reduction of skin oiliness (about 15 minutes);
- the invention provides a prolonged reduction and control of skin oiliness throughout the day;
- the invention is a prebiotic that restores balance of the skin microbiota;
- the invention minimizes the appearance of pores immediately (about 15 minutes) and in a prolonged manner;
- the invention improves the appearance and texture of skin with imperfection marks.

These and other objects of the invention will be immediately valued by those skilled in the art and by companies with interest in the segment, and will be described in sufficient detail to be reproduced in the description below.

### Brief description of the figures

In order to better define and clarify the present invention, the following figures are presented:
Figure 1 graphically represents the values of sebaceous secretion obtained in the study of the immediate effect (15 and 30 minutes) after application of the face mask of the invention.
Figure 2 graphically represents the values of sebaceous secretion obtained in the study of the prolonged effect (2, 4 and 8 hours) after application of the face mask of the invention.
Figure 3 graphically depicts the immediate effect (about 15 minutes of application) of reducing the average pore size when applying a face mask of the invention.
Figure 4 illustrates the immediate effect of reducing the average pore size after applying the face mask of the invention.
Figure 5 graphically represents the effect of the face mask of the invention on the initial skin color variation (prior to application of the mask) and after 30 days of use of the investigational product.

### Detailed description of the invention

The present invention describes a topical cosmetic composition and uses thereof in reducing and controlling oiliness, reducing pores, reducing visual skin imperfections, reducing shine and maintaining the skin's microbiota.

In the present invention, the term "maintenance of the skin microbiota" can also be understood as "preservation of the skin microbiota" or "microbiota balance", being defined as an ability to enable the presence of a natural and healthy skin microbiota. Furthermore, the term "skin microbiota" can also be understood as "skin flora".

In a first aspect the present invention relates to a topical cosmetic composition in the form of a clay face mask, wherein the composition comprises: clay, mandelic acid, salicylic acid and babassu starch.

By clay face mask is meant a cosmetically acceptable base formed from one or more types of clays. The mask comprises: clay, cosmetically acceptable active ingredients and excipients.

Among the required ingredients to provide the effects of the face mask of the present invention are mandelic acid, salicylic acid, clay and babassu starch. Preferably, the composition comprises about 0.1 to 8% by weight mandelic acid, about 0.1 to 2% by weight salicylic acid, about 2 to 40% by weight clay, and about 0.2 to 20% by weight babassu starch relative to the total weight of the composition.

In the present invention the clay can be a single type clay or a mixture of one or more types of clay. Examples of clay types are white, green, red, pink, grey, black clay, etc. Preferably, the clay is a mixture of black and green clays.

Preferably, the composition comprises from 1 to 20% black clay and from 1 to 20% green clay.

Mandelic acid is a high molecular weight alpha-hydroxy acid (2-hydroxy-2-phenylethanoic acid). Due to its high molecular weight, it is slowly absorbed by the skin, making a smoother exfoliation and stimulating cell renewal. On the skin, the compound improves the uneven texture of the skin resulting in an even appearance, in addition to leaving the skin softer and smoother.

Salicylic acid is a beta hydroxy acid (2-hydroxybenzoic acid). Salicylic acid acts as a keratolytic agent, cleaning clogged pores and preventing them from becoming clogged again. In addition, it acts in the reduction of dead cells and skin oil. Therefore, the compound promotes unclogging of pores and reduction in skin oiliness.

Babassu starch is a biotechnological active ingredient of natural origin that can be found in ingredients such as: Babassu coconut mesocarp flour (*Orbignya phalerata).* The ingredient acts in the maintenance and balance of skin microorganisms. On the skin it reduces the appearance of acne and comedones.

One or more clays used in the present invention are of the kaolin type.

In one embodiment of the first aspect, the composition is a clay face mask in the form of an emulsion, comprising active ingredients and cosmetically acceptable excipients.

By cosmetically acceptable excipients is meant nonirritating and non-toxic substances, which are used to prepare and/or provide structure to a cosmetic form, and may or may not confer organoleptic properties on a composition such as, for example, color and odor, and may or may not provide a cosmetically desirable biological effect such as hydration. The cosmetically acceptable excipients of the present invention can be formulated in different amounts, depending on the type of characteristics envisioned in the final cosmetic product.

The cosmetically acceptable excipients in accordance with the present invention may be those known to the person skilled in the art, such as those cited in the "International Cosmetic Ingredient Dictionary & Handbook", 16th Edition.

In a particular embodiment, the cosmetically acceptable excipients of the present invention can be, in a non-exhaustive manner, those belonging to the classes of solvents, emollients, absorbents, emulsifiers, opacifiers, skin conditioners, preservatives, viscosity controllers, skin protectors, denaturants, perfumes, moisturizers, antioxidants, emulsion stabilizers, plasticizers, chelating agents, dyes and buffers.

In a preferred embodiment, the present invention is a creamy emulsion containing active ingredients such as salicylic acid, mandelic acid, green and black clays and babassu starch, which promote pore unclogging and reduce oiliness through mechanisms of absorption, exfoliation and cell renewal.

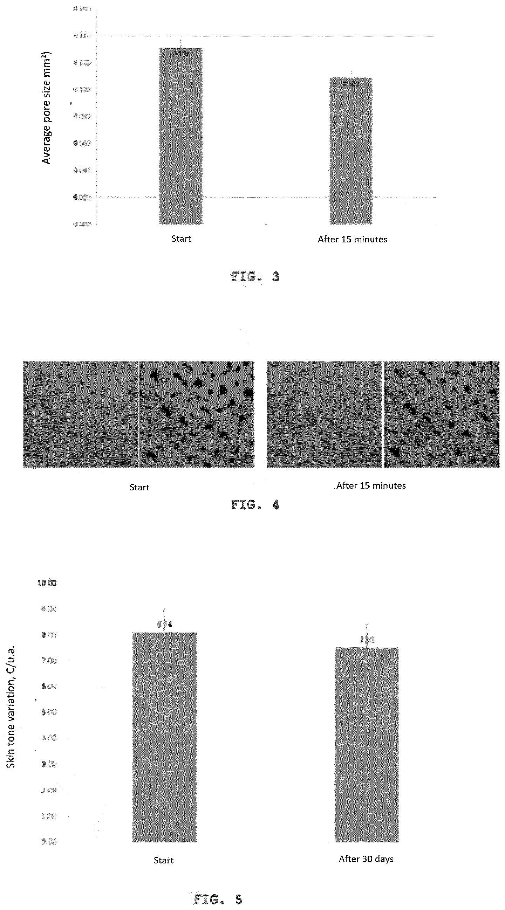

The present invention differs in many ways from the state of the art. For example, the preferred embodiment of the present invention is an emulsion, which is technically difficult to obtain due to mandelic acid instability. Moreover, the present invention exhibits an immediate (about 15 minutes after application of the product) and prolonged effect of reducing and controlling skin oiliness. It should be highlighted that the present invention provides an immediate effect on the control of oiliness, as shown in example 2 and figure 3.

The face mask of the present invention is applied to the skin so as to form an even layer of the product on the previously clean and dry face. Leave it to act for about 10 to 25 minutes and rinse with cold water. Preferably, the mask of the present invention is stays until it dries on the skin.

Among the technical effects of the first aspect are the immediate effect (about 15 minutes) in modulating the skin appearance, especially in terms of oiliness and reduction of pores size. An immediate reduction in oiliness by up to 60% is obtained. Intensive, fast-acting pore treatment: immediate reduction of the pore size by up to 25% - ¼ of seemingly less visible pores.

The first aspect of the invention is intended for consumers who have combined to oily skin and wish to complement their cosmetic treatment with regard to oil control and pore size reduction.

Thus, a second aspect of the present invention relates to the use of the composition to reduce and control skin oiliness.

In one embodiment the second aspect of the invention relates to an immediate reduction and control of skin oiliness.

By immediate is meant the time elapsed of about 15 minutes after application of the composition of the first aspect. One of the main aspects are: reduction of skin oiliness; progressive reduction of sebum secretion profile.

In another embodiment the second aspect of the invention relates to a prolonged reduction and control of skin oiliness.

In a third aspect, the present invention relates to the use of the composition to reduce the average size of skin pores.

In a fourth aspect, the present invention relates to the use of the composition to reduce visual skin imperfections.

Improvement of the general texture and uniformity conditions of the skin tone after continuous use of the product was evaluated through an objective non-invasive methodology designated as Image Analysis. The following time points were considered as the evaluation criterion after application of the product: T0; and T28 days after use of the product. Twenty-three participants were included with a metric premise for completion. The achieved results have shown that the composition of the first aspect reduces imperfections and provides a more uniform skin.

### Examples - Embodiments

The examples shown herein are intended only to exemplify one of the numerous ways of carrying out the invention, without limiting its scope.

For the purpose of illustrating the technical effects of the present invention, and not to limit it, whenever the examples refer to an "investigational product" reference will be made to a face mask, which is a preferred embodiment of the composition of the first aspect. The composition of the "investigational product" can be found in table 1.

**Table 1 - Composition of the investigational product**

| Ingredient | Concentration (% w/w) |
|---|---|
| Water | qsp.100 |
| Neutral Hydrated Ethyl Alcohol | 10 |
| Glycerin | 8 |
| Black clay (kaolin) | 6 |
| Green clay (kaolin) | 4 |
| Titanium dioxide | 5 |
| Propanediol | 4 |
| Palmitic acid | 4 |
| Silica | 3 |
| Tapioca Starch | 3 |
| Glyceryl dipalmitate | 2 |
| Babassu coconut mesocarp flour (*Orbignya phalerata)* | 2 |
| Cupuaçu butter | 1 |
| Glyceryl caprylate | 0.8 |
| Cetearyl alcohol | 0.8 |
| Sorbitan stearate | 0.7 |
| Perfume | 0.7 |
| Xanthan Gum | 0.5 |
| Salicylic acid | 0.5 |
| Mandelic Acid | 0.51 |

### Example 1 - Immediate effect (15 to 30 minutes) of the investigational product on the reduction and control of skin oiliness.

### Goal

This example is intended to demonstrate the immediate effect of the investigational product on the reduction and control of skin oiliness. Methodology

Twenty-three participants with a mean age of 40±11 were selected. Among the participants, the phototype (Fitzpatrick) distribution was as follows: 87% phototype III and 13% phototype IV. Participants were instructed to discontinue the use of any cosmetic products on the face 72 hours prior to the start of the study. There were no reports or evidence of adverse reactions during the study.

In the frontal region (forehead) of the participants, two sites were marked (areas measuring 2.5 cm x 4.0 cm). After 30 minutes of the participant staying in an air-conditioned environment, the initial data on oiliness was obtained at the marked sites.

Then, the procedure of cleaning the sites with water and neutral soap was performed. After 2 hours of cleaning, new measurements of skin oiliness were performed to collect baseline oiliness values. After these 2 hours the investigational product was applied and new measurements of skin oiliness were obtained after 15 and 30 minutes of application of the product.

After applying the investigational product, it was allowed to completely dry for 20 minutes on the face. After this time, a film formed on the skin face and it was gently removed by rinsing the face with water in a standardized manner.

Cleaning of the sites and application of the investigational product were made in a standardized manner for comparison purposes. Moreover, the environment remained controlled at all stages.

Measurements were made by placing a probe to measure the oil content by means of a photometric method (using Cassette Sebumeter SM810), keeping the pressure constant for 30 seconds. Reduction in skin oiliness is evidenced by the decreased amount of sebaceous secretion, the lower the value, the greater the reduction in oiliness, and the longer this reduction lasts, the greater the control of oiliness provided.

### Results

As a result, the investigational product was found to provide a significant reduction in face skin oiliness after 15 and 30 minutes of application, relative to the baseline condition (2 hours after cleaning the site with water and neutral soap). It shows that the investigational product provides reduced and controlled facial skin oiliness in as little as about 15 minutes.

The investigational product was also found to significantly improved performance as compared to the control, that is, skin cleaned with a cotton pad moistened with water.

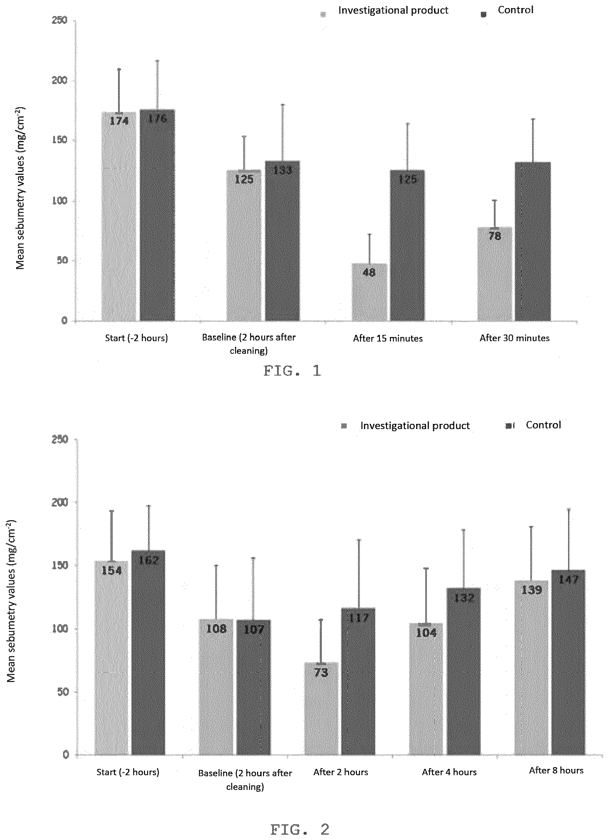

Figure 1 graphically represents the results achieved in this study. Figure 3 shows that the investigational product provided a 61.9% reduction in skin oiliness in about 15 minutes and 37.5% after 30 minutes of application.

### Example 2 - Prolonged effect of the investigational product on the reduction and control of skin oiliness.

### Goal

This example is intended to demonstrate the effectiveness in reducing and controlling facial skin oil after application of the investigational product. Methodology

Twenty-three participants with a mean age of 40±11 were selected. Among the participants, the phototype (Fitzpatrick) distribution was as follows: 87% phototype III and 13% phototype IV. Participants were instructed to discontinue the use of any cosmetic products on the face 72 hours prior to the start of the study. There were no reports or evidence of adverse reactions during the study.

In the frontal region (forehead) of the participants, two sites were marked (areas measuring 2.5 cm x 4.0 cm). After 30 minutes of the participant staying in an air-conditioned environment, the initial data on oiliness was obtained at the marked sites.

Then, the procedure of cleaning the sites with water and neutral soap was performed. After 2 hours of cleaning, new measurements of skin oiliness were performed to collect baseline oiliness values. After these 2 hours the investigational product was applied, and new measurements of skin oiliness were obtained after 2 and 4 hours.

After applying the investigational product, it was allowed to completely dry for 20 minutes on the face. After this time, a film formed on the skin face and it was gently removed by rinsing the face with water in a standardized manner.

Cleaning of the sites and application of the investigational product were made in a standardized manner for comparison purposes. Moreover, the environment remained controlled at all stages.

Measurements were made by placing a probe to measure the oil content by means of a photometric method (using Cassette Sebumeter SM810), keeping the pressure constant for 30 seconds. Reduction in skin oiliness is evidenced by the decreased amount of sebaceous secretion, the lower the value, the greater the reduction in oiliness, and the longer this reduction lasts, the greater the control of oiliness provided.

### Results

The investigational product was found to provide a significant reduction in face skin oiliness after 2 and 4 hours of application, relative to the baseline condition (2 hours after cleaning the site with water and neutral soap). It shows that the investigational product provides reduced and controlled facial skin oiliness for up to 4 hours.

Furthermore, the investigational product was found to presents a significantly improved performance over the control (skin with no products applied).

Figure 2 graphically represents the results achieved in this study. Figure 2 demonstrates that the investigational product was shown to provide a significant mean reduction of 31.9% in facial skin oiliness after 2 minutes of application relative to the baseline condition.

### Example 3 - Effects of the investigational product on the reduction of facial skin pore size by image analysis.

### Goal

This example is intended to demonstrate the effect of the investigational product on the issue of the reduction in pore size after and 8 hours of application of the product.

### Methodology

Twenty-three participants with a mean age of 40±11 were selected. Among the participants, the phototype (Fitzpatrick) distribution was as follows: 87% phototype III and 13% phototype IV. Participants were instructed to discontinue the use of any cosmetic products on the face 72 hours prior to the start of the study. There were no reports or evidence of adverse reactions during the study.

After applying the investigational product, it was allowed to completely dry for 20 minutes on the face. After this time, a film formed on the skin face and it was gently removed by rinsing the face with water in a standardized manner.

The results achieved are a comparison between high resolution photographic images under controlled and standardized conditions. Data were obtained at an initial time (prior to application of the product) and 15 minutes after applying the face mask.

According to each volunteer's face morphology, a selection area was determined for analysis of the apparent pore average size. Analysis was kept constant for each participant in terms of size and placement between the evaluation conditions, thus allowing a comparison to be made between the images.

The selected areas were assessed using a particle morphology analysis tool, which identifies, isolates and analyzes via color contrasting information the geometric parameters of the detected visible pores, as shown in Figure 4.

### Results

An immediate effect of the product in the reduction of the pore size was noticed, that is, after only 15 minutes. The observed values show that there was a significant reduction of 16.7% (P<0.05) in the average pore size, reaching up to 20.1%. The observed technical effect is shown in Figure 3 and Figure 4, which visually illustrate the immediate result (after 15 minutes of product application) of one of the participants studied.

### Example 4 - Effect of the investigational product in masking imperfections.

For the study, 10 participants were selected with a mean age of 32±9 years. 100% of the participants have phototype III (Fitzpatrick). The participants were instructed to discontinue the use of any topical product 48 hours prior to the start of the study.

The methodology consisted of the objective analysis performed by obtaining photographic images of the face of the research participants at the beginning of the study and after 30 days of home use of the investigational product.

As a result, after 30 days of use of the investigational product the skin color was found to be 7.7% more homogeneous, reaching up to 12.0%. Also, 90% of the research participants showed improved skin color homogenization after 30 days of use of the investigational product. Therefore, the product masked facial skin imperfections. Figure 5 shows the results graphically.

Those skilled in the art will value the knowledge presented herein and may reproduce the invention in the disclosed embodiments and other variants, as covered by the scope of the appended claims.

## Claims

1. A topical cosmetic composition **characterized by** comprising: 1 to 40% by weight clay, 0.1 to 8% by weight mandelic acid, 0.1 to 2% by weight salicylic acid and 0.2 to 20% by weight babassu starch, based on the total weight of the composition.

2. The composition according to claim 1, **characterized in that** the clay is a mixture of green clay and black clay.

3. The composition according to any one of claims 1 to 2, **characterized in that** the composition comprises 1 to 20% by weight of black clay and 1 to 20% by weight of green clay, relative to the total weight of the composition.

4. The composition according to any one of claims 1 to 3, **characterized in that** it is an emulsion.

5. The composition according to any one of claims 1 to 4, **characterized in that** it contains cosmetically acceptable excipients selected from the class of: solvents, emollients, absorbents, emulsifiers, opacifiers, skin conditioners, preservatives, viscosity controllers, skin protectors, denaturing agents, perfumes, moisturizers, antioxidants, emulsion stabilizers, plasticizers, chelating agents, dyes and buffers.

6. A topical cosmetic composition **characterized by** being a face mask.

7. Use of the composition as defined in any one of claims 1 to 6, **characterized in that** it is to reduce and control skin oiliness.

8. The use according to claim 7, **characterized in that** it is to immediately reduce and control the skin oiliness.

9. The use according to claim 7, **characterized in that** it is to provide prolonged reduction and control of skin oiliness.

10. The use of the composition as defined in any one of claims 1 to 7, **characterized in that** it is to reduce the average skin pore size.

11. The use of the composition as defined in any one of claims 1 to 7, **characterized in that** it is to reduce visual skin imperfections.

12. The use of the composition as defined in any one of claims 1 to 7, **characterized in that** it is to maintain the skin microbiota.

13. A face mask **characterized in that** it comprises the composition as defined in any one of claims 1 to 7.
